# EUROPEAN PATENT APPLICATION

(11) **EP 3 388 036 A1**
(43) Date of publication of application: **17.10.2018**
(21) Application number: 18167248.6
(22) Date of filing: 13.04.2018
(51) Int. Cl.: A61F 9/06, G09B 5/00, G09B 19/00, G09B 19/24

(54) **METHODS AND SYSTEMS FOR WIRELESS LIVE VIDEO STREAMING FROM A WELDING HELMET**

(30) Priority: 13.04.2017 US 201715486363
(71) Applicant: Lincoln Global, Inc., Santa Fe Springs, CA 90670 (US)
(72) Inventor: Cross, Charles, Euclid, OH Ohio 44132 (US); Relko, Michael, Wickliffe, OH Ohio 44092 (US)
(74) Representative: Grosse Schumacher Knauer von Hirschhausen

(57) **Abstract**

Embodiments of systems (100) and methods for wireless live video streaming from a welding helmet (110) are disclosed. In one embodiment, a welding helmet (110) includes an auto-darkening filter (210) having an inner surface. The welding helmet (110) also includes a camera (115) positioned next to the inner surface of the auto-darkening filter (210) at a location between two eyes of a user of the welding helmet (110), without obstructing a view of the user through the auto-darkening filter (210). The camera (115) is configured to generate image information in real time corresponding to a field-of-view through the auto-darkening filter (210) during a welding process. The welding helmet (110) further includes a video streaming device (120) operatively connected to the camera (115) and configured to receive the image information from the camera (115) and transmit the image information as real time live streaming video in a wireless radio frequency transmission.

## Description

### FIELD OF THE INVENTION

The invention is related to a welding training system according to claim 1, to a method to retrofit a welding helmet according to claim 11, and to a welding helmet according to claim 11. Embodiments of the present invention relate to systems and methods related to welding and cutting, and more specifically to capturing video via a welding helmet.

### TECHNICAL BACKGROUND

Conventionally, when a welding instructor or demonstrator gives an arc welding demonstration to customers or students, there are typically at least several people surrounding the welding location of a welding procedure being performed by the instructor or demonstrator. It is difficult for the surrounding people to see exactly what the instructor or demonstrator is viewing (e.g., the welding arc) as the welding procedure is being performed in real time. It is also difficult for the surrounding people to see the welding technique that is being used by the instructor or demonstrator, how the arc appears, what to do, what not to do, and arc and weld puddle characteristics from the view of the instructor or demonstrator.

### DESCRIPTION

In order to improve welding training, especially to simplify the efforts of a welding instructor, a welding training system according to claim 1 is described and a method to retrofit a welding helmet according to claim 7 and a welding helmet according to claim 11. Preferred embodiments are subject of the subclaims. One embodiment is a welding training system. The welding training system includes a welding helmet having an auto-darkening filter. The system also includes a camera positioned next to an inner surface of the auto-darkening filter within the welding helmet at a location between two eyes of a user of the welding helmet, without obstructing a view of the user of the welding helmet through the auto-darkening filter. The camera is configured to generate image information in real time corresponding to a field-of-view through the auto-darkening filter during a welding process. The system further includes a video streaming device integrated with the welding helmet. The video streaming device is operatively connected to the camera and is configured to receive the image information from the camera and transmit the image information as real time live streaming video in a wireless radio frequency transmission. The system also includes a mobile device configured to wirelessly receive the real time live streaming video in the radio frequency transmission, via an application running on the mobile device, and display the real time live streaming video to a user of the mobile device. The system further includes a monitoring device configured to receive the live streaming video from the mobile device and display the live streaming video to multiple human observers. In one embodiment, the system includes an adapter cable configured to be operatively connected between the mobile device and the monitoring device and mirror the real time live streaming video displayed on the mobile device to the monitoring device. In another embodiment, the monitoring device is configured to receive the live streaming video via a second wireless radio frequency transmission transmitted by the mobile device or by a wireless adapter operatively connected to the mobile device. The system may include a rechargeable power source integrated with the welding helmet. The rechargeable power source is operatively connected to the video streaming device and is configured to provide electrical power to the video streaming device. The system may include an antenna integrated with the welding helmet. The antenna is operatively connected to the video streaming device to facilitate transmitting of the wireless radio frequency transmission. In one embodiment, the application of the mobile device is configured to provide a graphical user interface to be displayed on the mobile device to allow a user of the mobile device to control at least one display feature of the real time live streaming video. The display feature may be, for example, a display brightness, a display contrast, a display zoom level, a display aspect ratio, or a display cropping. In one embodiment, the application of the mobile device is configured to provide a graphical user interface to be displayed on the mobile device to allow a user of the mobile device to select a control feature, causing the mobile device to wirelessly transmit a control signal in response to user selection of the control feature. The video streaming device of the welding helmet is configured to wirelessly receive the control signal and adjust a camera feature of the camera in response to the control signal. The camera feature may be, for example, a camera zoom or a camera focus.

One embodiment includes a method to retrofit a welding helmet. The method includes mounting a camera within a welding helmet next to an inner surface of an auto-darkening filter of the welding helmet. The camera is positioned at a location corresponding to a position between two eyes of a user of the welding helmet, without obstructing a view of the user of the welding helmet through the auto-darkening filter. The camera provides a field-of-view through the auto-darkening filter during a welding process. The method also includes mounting a video streaming device within the welding helmet and electrically connecting the video streaming device to the camera to receive video information from the camera. The method further includes mounting a rechargeable power source within the welding helmet and electrically connecting the rechargeable power source to the video streaming device to provide electrical power to at least the video streaming device. In one embodiment, the method includes mounting an antenna to the welding helmet and electrically connecting the antenna to the video streaming device to support wireless radio frequency transmission of the video information by the video streaming device. The antenna may be mounted on one of an inner surface or an outer surface of the welding helmet. Mounting of the camera, the video streaming device, the rechargeable power source, or the antenna may be accomplished via, for example, an adhesive material, a bracket, or a hook-and-loop fastener.

One embodiment includes a welding helmet. The welding helmet includes an auto-darkening filter having an inner surface. The welding helmet also includes a camera positioned next to the inner surface of the auto-darkening filter at a location between two eyes of a user of the welding helmet, without obstructing a view of the user of the welding helmet through the auto-darkening filter. The camera is configured to generate image information in real time corresponding to a field-of-view through the auto-darkening filter during a welding process. The welding helmet further includes a video streaming device operatively connected to the camera and configured to receive the image information from the camera and transmit the image information as real time live streaming video in a wireless radio frequency transmission. In one embodiment, the welding helmet includes a rechargeable power source operatively connected to the video streaming device and configured to provide electrical power to at least the video streaming device. In one embodiment, the welding helmet includes an antenna operatively connected to the video streaming device to facilitate transmitting of the wireless radio frequency transmission. The camera may provide a video frame rate of at least 24 frames per second or of at least 60 frames per second, in accordance with various embodiments (e.g., 100 frames per second). In accordance with some embodiments, the wireless radio frequency transmission is one of a Wi-Fi transmission or a Bluetooth® transmission.

Numerous aspects of the general inventive concepts will become readily apparent from the following detailed description of exemplary embodiments, from the claims, and from the accompanying drawings.

### BRIEF DESCRIPTION OF THE DRAWINGS

The accompanying drawings, which are incorporated in and constitute a part of the specification, illustrate various embodiments of the disclosure. It will be appreciated that the illustrated element boundaries (e.g., boxes, groups of boxes, or other shapes) in the figures represent one embodiment of boundaries. In some embodiments, one element may be designed as multiple elements or that multiple elements may be designed as one element. In some embodiments, an element shown as an internal component of another element may be implemented as an external component and vice versa. Furthermore, elements may not be drawn to scale.
FIG. 1 illustrates a system block diagram showing an embodiment of a system providing wireless live video streaming from a welding helmet;
FIG. 2 illustrates a view of an embodiment of the welding helmet of the system of FIG. 1;
FIG. 3 illustrates a view of an embodiment of a portion of the system of FIG. 1;
FIG. 4 illustrates a view of an embodiment of the system of FIG. 1 during a demonstration of the system; and
FIG. 5 illustrates a flowchart of an embodiment of a method for retrofitting a welding helmet to provide wireless live video streaming.

### DETAILED DESCRIPTION

Embodiments of the present invention include systems and methods related to live video streaming from a welding helmet. In one embodiment, a wireless camera (e.g. a high definition camera) is positioned inside an auto-darkening welding helmet. The camera is positioned directly between the eyes of the instructor or demonstrator on the inside lens of the auto-darkening helmet. The camera is small enough such that it is not noticed by the instructor/demonstrator (or at least does not obstruct the view of the instructor/demonstrator) when wearing the helmet. The camera indirectly and wirelessly provides video to an application running on a computerized mobile device (e.g. a mobile phone or tablet). That is, live video from the camera in the helmet is streamed to the mobile device and may be displayed on the mobile device. The mobile device is connected wirelessly (or in a wired manner) to a television screen or monitor. The wireless connection (or the wired connection) between the mobile device and the screen or monitor mirrors what is displayed on the mobile device to the screen or monitor.

In one embodiment, the application on the mobile device allows a user of the mobile device to control aspects of the displayed video (e.g., aspect ratio, cropping, brightness, contrast, other display parameters) displayed on the mobile device (and, therefore, on the screen or monitor) via the mobile device. In another embodiment, the application on the mobile device allows a user of the mobile device to control aspects of the camera (e.g., zoom, focus, other camera parameters). Since the mobile device is connected to the screen or monitor, more people can see close up what the instructor or demonstrator is viewing through the auto-darkening lens of the welding helmet when making a weld. Such an embodiment may also be used in plasma cutting applications.

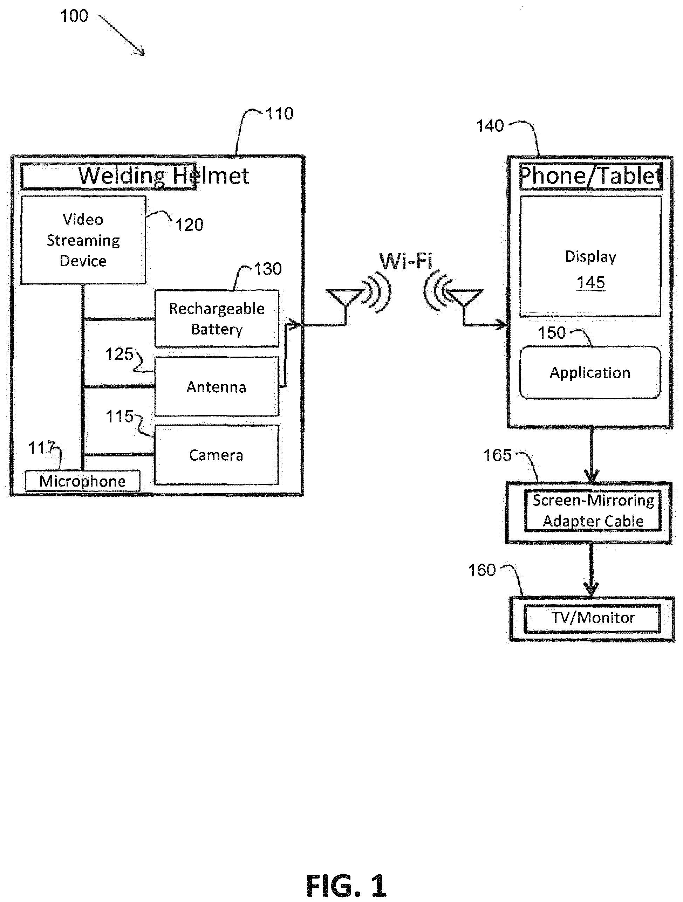

The examples and figures herein are illustrative only and are not meant to limit the subject invention, which is measured by the scope and spirit of the claims. Referring now to the drawings, wherein the showings are for the purpose of illustrating exemplary embodiments of the subject invention only and not for the purpose of limiting same, FIG. 1 illustrates a system block diagram showing one embodiment of a system 100 providing wireless live video streaming from a welding helmet.

The system 100 of FIG. 1 includes a welding helmet 110 having a camera 115, a video streaming device 120, an antenna 125, and a rechargeable power source 130. The system 100 also includes a mobile device 140 having a display 145 and an application 150. The application 150 includes computer-executable instructions that are stored in a memory (not shown) of the mobile device 140. The computer-executable instructions are capable of being executed by a processor (not shown) of the mobile device 140. The system further includes a television or monitor 160 (referred to hereinafter as a monitor or monitoring device) and an adapter cable 165.

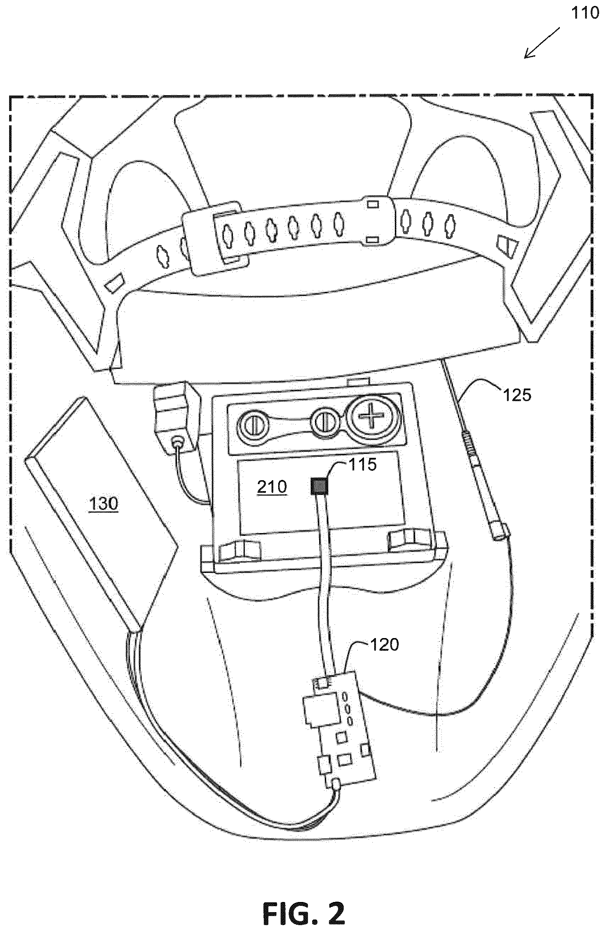

FIG. 2 illustrates a view of an embodiment of the welding helmet 110 of the system 100 of FIG. 1. In the view of FIG. 2, the camera 115, the video streaming device 120, the antenna 125, and the rechargeable power source 130 are shown. The welding helmet 110 also includes an auto-darkening filter 210 which protects the eyes of a welder from the bright light produced by a welding arc. The auto-darkening filter automatically switches between a darkened protective state (when an arc is present) and an un-darkened state (when an arc is not present). In accordance with an alternative embodiment, the auto-darkening filter is replaced by a dark filter which does not switch darkening states and is permanently dark.

In one embodiment, the camera 115 is positioned next to an inner surface or lens of the auto-darkening filter 210 within the welding helmet 110. The camera 115 is positioned at a substantially central position, corresponding to a location between two eyes of a user of the welding helmet 110, such that the view of the user through the auto-darkening filter is not obstructed by the camera 115. The camera 115 is capable of generating image information in real time corresponding to a field-of-view through the auto-darkening filter during a welding process. In this manner, the camera 115 "sees" substantially the same view (or at least a substantial portion of the view) that is seen by the welder wearing the welding helmet 110.

In one embodiment, the camera 115 provides color image data and in another embodiment the camera provides gray-scale image data. In still another embodiment, the camera may be configured to be switched between color and gray-scale modes of operation. The video frame rate of the camera 115 is 24 frames per second or greater, in accordance with one embodiment. The video frame rate of the camera 115 is 60 frames per second or greater, in accordance with another embodiment. Higher frames rates allow observers to see more real time characteristics of, for example, the arc and the weld puddle during a welding procedure. In one embodiment, the camera 115 provides high definition video resolution (e.g., 1080P or 4K), allowing observers to see more detailed spatial and color characteristics of, for example, the arc and the weld puddle during a welding procedure.

In FIG. 2, the video streaming device 120 is attached to an inner surface of the welding helmet 110 at a position below the camera 115. The video streaming device may include a printed circuit board (PCB) with electronic components mounted thereon, in accordance with one embodiment. The video streaming device 120 is operatively connected to the camera 115 (e.g., via an electrical connector or cable) such that the camera 115 can provide image information to the video streaming device 120. The video streaming device 120 is capable of receiving the image information from the camera 115 and transmitting the image information as real time live streaming video in a wireless radio frequency transmission (signal) (e.g., a Wi-Fi transmission or a Bluetooth® transmission).

In FIG. 2, the antenna 125 is attached to an inner surface of the welding helmet 110 to the right of the camera 115. The antenna 125 is operatively connected (e.g., via an antenna cable) to the video streaming device 120 and facilitates transmitting of the wireless radio frequency transmission (signal). Also, in FIG. 2, the rechargeable power source 130 (e.g., a lithium battery) is attached to an inner surface of the welding helmet 110 to the left of the camera 115. The rechargeable power source 130 is operatively connected (e.g., via a power cable) to the video streaming device 120 to provide electrical power to at least the video streaming device 120. In one embodiment, the video streaming device 120 provides electrical power (originating at the power source 130) to the camera 115. In another embodiment, the power source 130 provides electrical power directly to the camera 115 (as well as directly to the video streaming device 120).

Although various elements of the welding helmet 110 are shown in FIG. 2 as being positioned and attached within the helmet 110, in other embodiments, the various elements may be more integral to the welding helmet 110. For example, the camera 110 may be an integral part of the auto-darkening filter 210. The antenna 125 may be integrated into an outer portion of the shell of the welding helmet 110. The video streaming device 120 and the rechargeable power source 130 may be integrated into an inner portion of the shell of the welding helmet 110. Other embodiments may provide compartments within the welding helmet 110, allowing the various elements to be installed into and removed from the compartments. The electrical connections between the various elements may be integrated within the shell of the welding helmet 110, for example. Other integrated configurations are possible as well, in accordance with other various embodiments. Such integrated embodiments may lend themselves more to new welding helmet designs as opposed to the retrofitting of existing welding helmets.

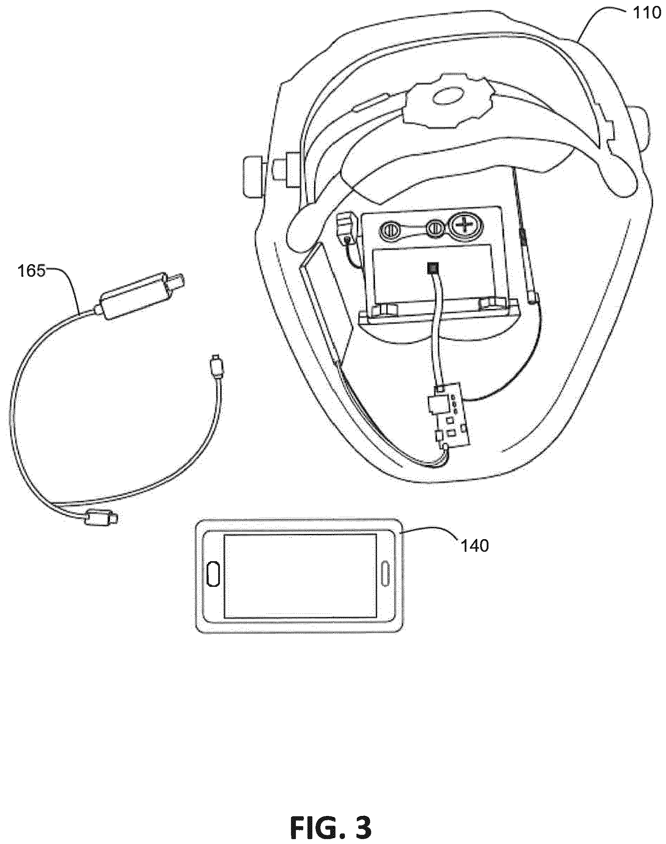

FIG. 3 illustrates a view of an embodiment of a portion of the system 100 of FIG. 1 including the welding helmet 110, the mobile device 140 (e.g., a smart phone or tablet computer), and the adapter cable 165. In one embodiment, the mobile device 140 is configured to wirelessly receive the real time live streaming video in the radio frequency transmission from the welding helmet via the application 150 running on the mobile device 140. The mobile device 140 is also configured to display the real time live streaming video to a user of the mobile device 140 on the display 145 of the mobile device 140. In an alternative embodiment, the mobile device 140 is replaced with, for example, a desktop computer or a work station that functions similarly to the mobile device 140. Such an alternative embodiment may be more appropriate for a dedicated training space, for example, having a fixed set up.

The adapter cable 165 is configured to be connected between the mobile device 140 and the monitor 160. In one embodiment, the adapter cable 165 is capable of converting digital streaming video out of a multi-pin port of the mobile device 140 to a high definition multi-media interface (HDMI) format that is fed into an HDMI port of the monitor 160. In this manner, the adapter cable 165 mirrors the real time live streaming video displayed on the mobile device 140 to the monitor 160. The real time live streaming video may be displayed on the monitor 160 and viewed by multiple human observers who may not be in a position to directly view the actual welding procedure. Furthermore, the multiple human observers can view the real time live streaming video of the welding procedure on the monitor 160 without having to wear protective gear such as, for example, a welding helmet. In accordance with one embodiment, the multi-pin port of the mobile device 140 is the same port that is used to charge the mobile device 140.

In accordance with one embodiment, in addition to the camera 115, a microphone 117 is provided within the welding helmet 110. The microphone 117 provides audio signals to the video streaming device 120 such that both video and audio are streamed via a wireless radio frequency transmission (signal). The audio may be heard, for example, via a speaker of the monitoring device 160. In this way, commentary of the instructor or demonstrator can be heard by the students during a demonstrated welding procedure.

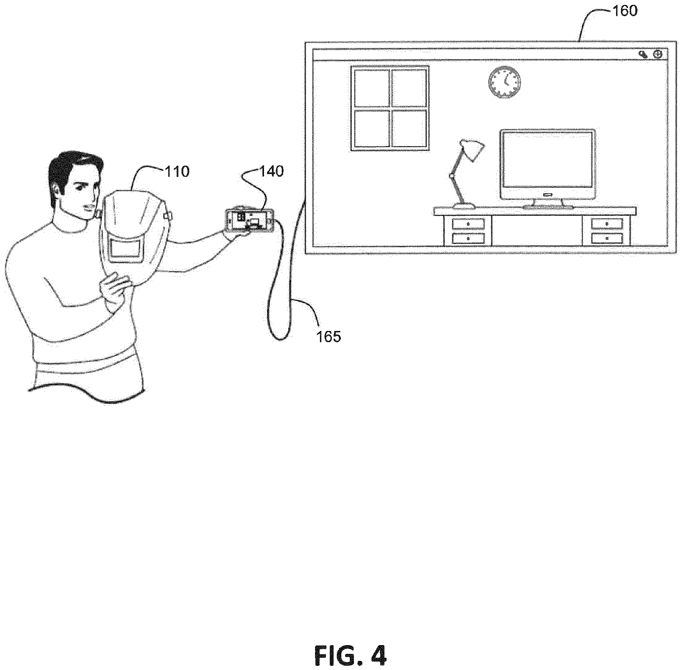

FIG. 4 illustrates a view of an embodiment of the entire system 100 of FIG. 1 (including the welding helmet 110, the mobile device 140, the adapter cable 165, and the monitor 160) during an office demonstration of the system 100. In this office demonstration, the auto-darkening filter 210 has been removed from the helmet 110. In FIG. 4, a human demonstrator is holding the welding helmet 110 (and the mobile device 140) and has the welding helmet 110 pointed toward a desk in an office. The camera 115 of the welding helmet 110 "sees" the desk, and the video streaming device 120 of the welding helmet 110 wirelessly transmits real time live streaming video of the desk to the mobile device 140. The real time live streaming video of the desk is displayed on the mobile device 140. Furthermore, the adapter cable 165 (connected between the mobile device 140 and the monitor 160) mirrors the real time live streaming video displayed on the mobile device 140 to the monitor 160. The monitor 160 is large compared to the mobile device and, therefore, more people are able to more easily view the video on the monitor 160 at the same time than on the mobile device 140.

In accordance with an alternative embodiment, the monitor 160 is configured to receive the live streaming video (and/or audio) via a second wireless radio frequency transmission (signal) transmitted by the mobile device 140. In this manner, the adapter cable 165 may be eliminated from the system 100. In yet another embodiment, the adapter cable 165 is configured as a wireless adapter operatively connected to the mobile device 140. The wireless adapter is configured to transmit a second wireless radio frequency transmission (signal) to provide the real time live streaming video from the mobile device 140 to the monitor 160. The monitor 160 is configured to receive the second wireless radio frequency transmission (signal). The wireless radio frequency transmission from the video streaming device may be on a different frequency and/or use a different transmission protocol so as not to interfere with the second wireless radio frequency transmission from the mobile device 140 or the wireless adapter. Furthermore, in a larger welding school environment, for example, the second wireless radio frequency transmission may be received by multiple monitors such that many more students can view a welding procedure live in real time.

In accordance with one embodiment, the application 150 of the mobile device 140 is programmed to provide a graphical user interface (GUI) to be displayed on the display 145 of the mobile device 140. The GUI is configured to aid a user of the mobile device 140 in wirelessly connecting to the video streaming device 120 of the welding helmet 110 (e.g., via Wi-Fi). In one embodiment, the GUI is also configured to allow a user of the mobile device 140 to control display features of the real time live streaming video. Such display features may include, for example, a display brightness, a display contrast, a display zoom level, a display aspect ratio, or a display cropping. Control of other display features may be possible as well, in accordance with other embodiments.

In accordance with another embodiment, the GUI is configured to allow a user of the mobile device 140 to select a control feature, causing the mobile device 140 to wirelessly transmit a control signal in response to the user selection. In such an embodiment, the video streaming device 120 of the welding helmet 110 is configured to wirelessly receive the control signal (e.g., via the antenna 125) and adjust a camera feature of the camera 115 in response to the control signal. Such camera features may include, for example, a camera zoom and a camera focus. Control of other camera features (or microphone features...e.g., volume) may be possible as well, in accordance with other embodiments.

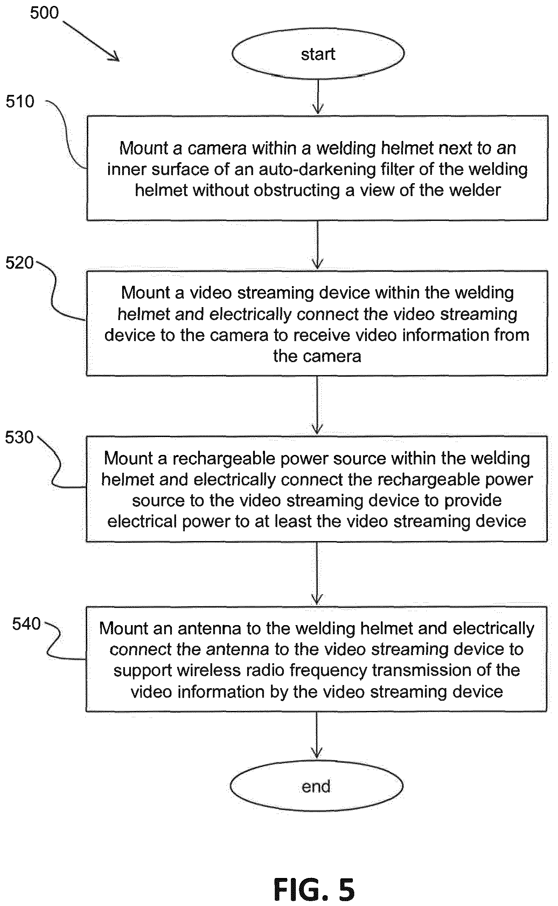

FIG. 5 illustrates a flowchart of an embodiment of a method 500 for retrofitting an existing welding helmet to provide wireless live video streaming. In one embodiment, the welding helmet to be retrofitted is assumed to have an auto-darkening filter. At block 510 of the method 500, a camera is mounted within the welding helmet next to an inner surface of the auto-darkening filter. The camera is substantially centrally positioned at a location corresponding to a position between two eyes of a user of the welding helmet, without obstructing a view of the user through the auto-darkening filter. In this manner, a field-of-view for the camera through the auto-darkening filter is provided, allowing the camera to "see", at least to a large extent, what the user sees.

At block 520, a video streaming device is mounted within the welding helmet and is electrically connected to the camera to receive video information from the camera. At block 530, a rechargeable power source is mounted within the welding helmet and is electrically connected to the video streaming device to provide electrical power to the video streaming device. In one embodiment, the video streaming device provides electrical power (derived from the rechargeable power source) to the camera.

At block 540, an antenna is mounted to the welding helmet and is electrically connected to the video streaming device to support wireless radio frequency transmission of the video information by the video streaming device. In one embodiment, the antenna is mounted to an inner surface of the welding helmet. In another embodiment, the antenna is mounted to an outer surface of the welding helmet. In accordance with an alternative embodiment, a small antenna is integrated in the video streaming device, thus eliminating the need to mount an antenna external to the video streaming device.

Mounting of any of the camera, the video streaming device, the rechargeable power source, or the antenna may be accomplished via, for example, one or more of adhesive materials, brackets, and/or hook-and-loop fasteners (e.g., Velcro®), in accordance with various embodiments. The electrical connections between the various elements may be accomplished via, for example, appropriate electrical connectors and/or cables (e.g., an antenna cable, a power cable, a video cable, etc.). The various connectors and/or cables may be positioned and held in place via, for example, adhesive materials, clamps, and/or hook-and-loop fasteners, in accordance with various embodiments.

In one embodiment, the video streaming device 120 includes an analysis portion that is capable of analyzing image data from the camera to determine characteristics of, for example, the arc, the weld puddle, stick out (etc.) and generating data representing such characteristics. Such characteristics may include, for example, arc on/off, arc length, arc voltage, liquid weld pool dimensions, heat-affected zone dimensions, fusion zone dimensions, etc. The data may be wirelessly streamed in real time, along with the video. In one embodiment, the application on the mobile device 140 is programmed to integrate the streamed data with the streamed video such that the data may be displayed with the video in real time. In another embodiment, the video streaming device in the welding helmet is configured to integrate the streamed data with the streamed video, before radio frequency transmission, such that the data may be displayed with the video in real time. In accordance with one embodiment, the analysis portion of the video streaming device 120 includes an analysis application running on a processor of the video streaming device 120.

In one embodiment, streamed data and video wirelessly transmitted from the video streaming device 140 is received by an external device (e.g., a server computer), recorded (e.g., in a memory of the server computer), and stored to a training database computer accessible via a computer network (e.g., a local area network (LAN), a wide area network (WAN), the internet). The streamed data and video may correspond to, for example, a welding session performed by a welding student. A welding instructor or the welding student can access the training database computer and review the video and data for educational purposes, for example, in accordance with one embodiment. Such a training database computer may be tied into a welding education curriculum, in accordance with one embodiment.

While the disclosed embodiments have been illustrated and described in considerable detail, it is not the intention to restrict or in any way limit the scope of the appended claims to such detail. It is, of course, not possible to describe every conceivable combination of components or methodologies for purposes of describing the various aspects of the subject matter. Therefore, the disclosure is not limited to the specific details or illustrative examples shown and described. Thus, this disclosure is intended to embrace alterations, modifications, and variations that fall within the scope of the appended claims, which satisfy the statutory subject matter requirements of 35 U.S.C. §101. The above description of specific embodiments has been given by way of example. From the disclosure given, those skilled in the art will not only understand the general inventive concepts and attendant advantages, but will also find apparent various changes and modifications to the structures and methods disclosed. It is sought, therefore, to cover all such changes and modifications as fall within the spirit and scope of the general inventive concepts, as defined by the appended claims, and equivalents thereof.

**REFERENCE NUMBERS**

| | | | |
|---|---|---|---|
| 100 | system | 160 | monitor |
| 110 | welding helmet | 165 | adapter cable |
| 115 | camera | 210 | auto darkening filter |
| 117 | microphone | 500 | method |
| 120 | video streaming device | 510 | block |
| 125 | antenna | 520 | block |
| 130 | power source | 530 | block |
| 140 | mobile device | 540 | block |
| 145 | display | | |
| 150 | application | | |

## Claims

1. A welding training system (100), comprising:
a welding helmet (110) having an auto-darkening filter (210);
a camera (115) positioned next to an inner surface of the auto-darkening filter (210) within the welding helmet (110) at a location between two eyes of a user of the welding helmet (110), without obstructing a view of the user of the welding helmet (110) through the auto-darkening filter (210), wherein the camera (115) is configured to generate image information in real time corresponding to a field-of-view through the auto-darkening filter (210) during a welding process;
a video streaming device (120) integrated with the welding helmet (110), wherein the video streaming device (120) is operatively connected to the camera (115) and is configured to receive the image information from the camera (115) and transmit the image information as real time live streaming video in a wireless radio frequency transmission;
a mobile device (140) configured to wirelessly receive the real time live streaming video in the radio frequency transmission, via an application (150) running on the mobile device (140), and display the real time live streaming video to a user of the mobile device (140); and
a monitoring device (160) configured to receive the live streaming video from the mobile device (140) and display the live streaming video to a plurality of human observers.

2. The welding training system (100) of claim 1, further comprising an adapter cable (165) configured to be operatively connected between the mobile device (140) and the monitoring device (160) and mirror the real time live streaming video displayed on the mobile device to the monitoring device (160), and/or further comprising a rechargeable power source (130) integrated with the welding helmet (110), wherein the rechargeable power source (130) is operatively connected to the video streaming device (120) and is configured to provide electrical power to at least the video streaming device (120).

3. The welding training system (100) of claim 1 or 2, wherein the monitoring device (160) is configured to receive the live streaming video via a second wireless radio frequency transmission transmitted by the mobile device (140) or by a wireless adapter operatively connected to the mobile device (140).

4. The welding training system (100) of any of the claims 1 to 3, further comprising an antenna (125) integrated with the welding helmet (110), wherein the antenna (125) is operatively connected to the video streaming device (120) to facilitate transmitting of the wireless radio frequency transmission.

5. The welding training system (100) of any of the claims 1 to 4, wherein the application of the mobile device (140) is configured to provide a graphical user interface to be displayed on the mobile device (140) to allow a user of the mobile device (140) to control at least one display feature of the real time live streaming video,
wherein, preferably, the at least one display feature includes at least one of a display brightness, a display contrast, a display zoom level, a display aspect ratio, or a display cropping.

6. The welding training system (100) ofany of the claims 1 to 5:
wherein the application (150) of the mobile device (140) is configured to provide a graphical user interface to be displayed on the mobile device (140) to allow a user of the mobile device to select a control feature, causing the mobile device (140) to wirelessly transmit a control signal in response to user selection of the control feature; and
wherein and the video streaming device (120) of the welding helmet (110) is configured to wirelessly receive the control signal and adjust a camera feature of the camera (115) in response to the control signal, wherein, preferably, the camera feature includes one of a camera zoom or a camera focus.

7. A method to retrofit a welding helmet, the method comprising:
mounting a camera within a welding helmet next to an inner surface of an auto-darkening filter of the welding helmet at a location corresponding to a position between two eyes of a user of the welding helmet, without obstructing a view of the user of the welding helmet through the auto-darkening filter, to provide a field-of-view for the camera through the auto-darkening filter during a welding process;
mounting a video streaming device within the welding helmet and electrically connecting the video streaming device to the camera to receive video information from the camera; and
mounting a rechargeable power source within the welding helmet and electrically connecting the rechargeable power source to the video streaming device to provide electrical power to at least the video streaming device.

8. The method of claim 7, further comprising mounting an antenna to the welding helmet and electrically connecting the antenna to the video streaming device to support wireless radio frequency transmission of the video information by the video streaming device, wherein the antenna is mounted on one of an inner surface or an outer surface of the welding helmet.

9. The method of claim7 or 8, wherein the mounting of at least one of the camera, the video streaming device, the rechargeable power source, or the antenna is accomplished via at least one adhesive material and/or
wherein the mounting of at least one of the camera, the video streaming device, the rechargeable power source, or the antenna is accomplished via at least one bracket.

10. The method of any of the claims 7 to 9, wherein the mounting of at least one of the camera, the video streaming device, the rechargeable power source, or the antenna is accomplished via at least one hook-and-loop fastener.

11. A welding helmet (110) comprising:
an auto-darkening filter (210) having an inner surface;
a camera (115) positioned next to the inner surface of the auto-darkening filter (210) at a location between two eyes of a user of the welding helmet (110), without obstructing a view of the user of the welding helmet (110) through the auto-darkening filter (210), wherein the camera (115) is configured to generate image information in real time corresponding to a field-of-view through the auto-darkening filter (210) during a welding process; and
a video streaming device (120) operatively connected to the camera (115) and configured to receive the image information from the camera (115) and transmit the image information as real time live streaming video in a wireless radio frequency transmission.

12. The welding helmet (110) of claim 11, further comprising a rechargeable power source (130) operatively connected to the video streaming device (120) and configured to provide electrical power to at least the video streaming device (120).

13. The welding helmet (110) of claim 11 or 12, further comprising an antenna (125) operatively connected to the video streaming device (120) to facilitate transmitting of the wireless radio frequency transmission.

14. The welding helmet (110) of any of the claims 11 to 13, wherein the camera (115) provides a video frame rate of at least 24 frames per second or wherein the camera (115) provides a video frame rate of at least 60 frames per second.

15. The welding helmet (110) of any of the claims 11 to 14, wherein the wireless radio frequency transmission is one of a Wi-Fi transmission or a Bluetooth® transmission.
